# FASCICULE DE BREVET EUROPEEN

(11) **EP 3 389 575 B1**
(45) Date de publication et mention de la délivrance du brevet: **06.11.2019**
(21) Numéro de dépôt: 16808817.7
(22) Date de dépôt: 05.12.2016
(51) Int. Cl.: A61F 5/44, A61M 1/00, A61F 5/445

(54) **DISPOSITIF PERMETTANT UN FLUX DE BOL ALIMENTAIRE ENTRE DEUX STOMIES**
VORRICHTUNG MIT EINEM NAHRHAFTEN BOLUSFLUSS ZWISCHEN ZWEI STOMATA
DEVICE ALLOWING AN ALIMENTARY BOLUS FLOW BETWEEN TWO STOMAS

(30) Priorité: 15.12.2015 FR 1562433
(43) Date de publication de la demande: 24.10.2018
(73) Titulaire: Centre Hospitalier Régional Universitaire de Lille, 59037 Lille Cedex (FR)
(72) Inventeur: LOGIER, Régis, 59520 Marquette-les-Lille (FR); SOZANSKI, Jean-Pierre, 59650 Villeneuve d'Ascq (FR); NZAMUSH ELEPAN MABLA, Jean-Robert, 1420 Braines l'Alleud (BE)
(74) Mandataire: Schwalek, Valérie
(86) Numéro de dépôt international: PCT/IB2016/057348
(87) Numéro de publication internationale: WO 2017/103727

(56) Documents cités:
- WO-A1-2009/046995
- WO-A1-2014/122378
- US-A1- 2013 304 231

## Description

La présente demande concerne un dispositif permettant un flux de bol alimentaire entre deux stomies du type comportant des moyens anti-reflux comprenant un ballonnet présentant un col pour le passage du bol alimentaire.

Le document WO 2014/122378 décrit un dispositif permettant de faire circuler un flux de bol alimentaire entre deux stomies. Ce dispositif comporte un conduit d'aspiration et un conduit de refoulement. Le bol alimentaire provenant d'une première stomie est éjecté naturellement vers le collecteur du dispositif précité. Le conduit d'aspiration permet l'aspiration du bol alimentaire contenu dans le collecteur vers le conduit d'aspiration du dispositif qui permet, lui, de refouler le bol alimentaire aspiré dans la deuxième portion d'intestin au niveau de la deuxième stomie. Dans le document précité, les conduits d'aspiration et de refoulement sont chacun équipé d'une valve anti-retour. Cette valve anti-retour peut être du type bec de canard c'est-à-dire formée de deux lèvres souples susceptibles de s'écarter l'une de l'autre de manière à laisser passer un flux de bol alimentaire dans un seul sens. Les valves anti-retour peuvent également être de simples volets montés mobiles dans le conduit considéré de manière à permettre le passage du bol alimentaire dans un seul sens.

Il s'est avéré que les deux types de valve anti-retour ou moyens anti-reflux précités n'étaient pas adaptés au bol alimentaire. En effet, celui-ci contient des morceaux solides qui sont susceptibles de se bloquer entre les deux lèvres de la valve type bec de canard. De même, ces morceaux solides restent souvent bloqués entre le volet et la paroi du conduit empêchant le changement de position du volet et bouchant partiellement le conduit lui-même.

Par ailleurs, l'étanchéité des deux valves anti-retour précitées n'est pas satisfaisante. La mauvaise étanchéité peut même empêcher tout pompage du bol alimentaire voire engendrer un flux de bol alimentaire dans le sens inverse de celui souhaité.

Un but de la présente invention est de remédier à au moins un des problèmes précités.

La présente invention propose ainsi un dispositif permettant de créer un flux de bol alimentaire entre deux stomies, du type comportant :
- des moyens formant pompe qui présentent une ouverture d'aspiration et une ouverture de refoulement, qui sont adaptés pour aspirer un bol alimentaire à travers ladite ouverture d'aspiration et le refouler à travers ladite ouverture de refoulement, qui sont externes au corps du patient et aptes à être montés sur le corps d'un patient,
- des premiers moyens de connexion étanche, aptes à relier ladite ouverture d'aspiration à une stomie amont, située sur la paroi abdominale d'un patient ;
- des deuxièmes moyens de connexion étanche, aptes à relier ladite ouverture de refoulement à une stomie aval, disposée sur la paroi abdominale dudit patient ; et
- des premiers et/ou deuxièmes moyens anti-reflux aptes à éviter le reflux du bol alimentaire aspiré ou refoulé par lesdits moyens formant pompe vers ladite ouverture d'aspiration et/ou de refoulement.

Selon l'invention, de manière caractéristique, lesdits premiers et/ou deuxièmes moyens anti-reflux comportent :
- une cavité dans laquelle transit ledit bol alimentaire ;
- un ballonnet gonflable monté dans ladite cavité ; et
- des moyens de gonflage dudit ballonnet qui permettent de modifier le gonflement de ce dernier, ledit ballonnet est conformé pour passer d'une position d'ouverture dans laquelle ledit ballonnet est partiellement gonflé et présente un col qui permet le passage du bol alimentaire, à une position de fermeture dans laquelle ledit ballonnet est davantage gonflé que dans ladite position d'ouverture et dans laquelle ledit ballonnet est au contact de la paroi de ladite cavité et ledit col est obturé, notamment du fait de l'écrasement de la portion de paroi dudit ballonnet définissant ledit col sur elle-même, sur au moins une portion de la hauteur dudit col.

Le col du ballonnet permet de former un passage plus large que celui des valves de type bec de canard. Le gonflage et le dégonflage du ballonnet permettent de contrôler facilement la taille du col et donc le flux de bol alimentaire.

L'obturation du col est obtenue par rétrécissement de ce dernier puis contact de la portion de paroi du ballonnet définissant ledit col avec elle-même, sur au moins une portion de la hauteur du col.

Le ballonnet peut également servir facilement à la détection de la présence de bol alimentaire dans le dispositif de l'invention.

Le montage du ballonnet dans la cavité n'est pas limité selon l'invention. Ainsi, lorsque le ballonnet est en position d'ouverture, le bol alimentaire peut passer à travers le col uniquement ou à travers le col et entre la paroi de la cavité et le ballonnet.

Avantageusement, ledit ballonnet est monté dans ladite cavité de manière à ce que dans ladite position d'ouverture, la paroi dudit ballonnet reste en contact avec la paroi de ladite cavité, moyennant quoi le bol alimentaire circule uniquement à travers ledit col. On libère ainsi un passage de grande taille pour le transit du bol alimentaire ; le col formé par le ballonnet atteint ainsi sa taille maximale en position d'ouverture et le ballonnet n'a pas de risque d'être arraché au passage du bol alimentaire.

La forme du ballonnet n'est pas limitée selon l'invention dès qu'il présente un col qui peut se déformer pour se fermer par contact lorsque le ballonnet dépasse un état de gonflage donné. Le ballonnet peut être sensiblement cylindrique avec un passage sensiblement cylindrique et central qui définit le col. Il peut également être sensiblement annulaire ou sensiblement torique (en forme de beignet).

Selon l'invention, la forme de la cavité et/ou celle du ballonnet ne sont pas limitées. La cavité peut présenter sensiblement la même section que ledit conduit d'aspiration ou de refoulement et être une portion dudit conduit d'aspiration ou de refoulement. La cavité peut présenter une section de dimension égale ou supérieure à la section dudit conduit d'aspiration ou de refoulement. La cavité peut présenter une section circulaire ou elliptique dans un plan perpendiculaire à la longueur du conduit et/ou dans un plan parallèle à la longueur du conduit considéré.

Le ballonnet peut être monté dans la cavité au moyen d'un support ou directement sur la paroi de cette dernière. Il peut être collé sur la paroi de la cavité, par exemple. Le ballonnet peut être monté directement ou indirectement sur ladite paroi de ladite cavité. Le ballonnet est ainsi formé dans la cavité ce qui évite le passage du bol alimentaire entre la paroi de la cavité et le ballonnet, même en position d'ouverture.

Selon un mode de réalisation préféré, les moyens anti-reflux comportent, en outre, une bague qui comporte ladite cavité et une bande en matériau élastiquement déformable fixée de manière étanche sur la paroi de ladite cavité et formant ledit ballonnet gonflable. Cette bague peut être montée de manière étanche dans le conduit de refoulement/d'aspiration.

Selon un autre mode de réalisation, le ballonnet peut présenter une forme torique et sa surface externe est collée sur la bague, à l'intérieur de la cavité.

Selon un autre mode de réalisation le ballonnet est cylindrique et ses deux extrémités sont reliées l'une à l'autre ; il forme ainsi une couronne ou tore.

Avantageusement, ladite bague comporte une perforation pour le passage d'un fluide provenant desdits moyens de gonflage du ballonnet et une gorge creusée dans l'épaisseur de la paroi de ladite bague et qui débouche au niveau de ladite perforation. La gorge permet d'éviter que la membrane formant le ballonnet ne se colle contre la paroi de la cavité dans la position d'ouverture ou même soit aspirée par les moyens formant pompe. Cette gorge permet le passage du fluide (air ou autre gaz, voire liquide) venant des moyens formant pompe et sa répartition dans tout le volume du ballonnet.

Avantageusement, lesdits premiers et/ou deuxièmes moyens anti-reflux font partie desdits premiers moyens de connexion étanche et/ou desdits deuxièmes moyens de connexion étanche.

Selon un mode de réalisation particulier des moyens formant pompe qui n'est pas forcément combiné aux moyens anti-reflux précités, ceux-ci comportent une membrane déformable formant une coupelle ladite membrane est apte à passer de manière réversible d'une première position dans laquelle la première face de ladite membrane forme la surface interne concave de ladite coupelle à une deuxième position dans laquelle ladite première face forme la surface externe convexe de ladite coupelle.

Le dispositif peut également comporter des moyens de détection de la présence de bol alimentaire dans lesdits premiers moyens de collecte et notamment des moyens de détection de la présence de bol alimentaire qui comportent des moyens de détection d'une surpression dans le ballonnet équipant lesdits premiers moyens anti-reflux

Selon l'invention, le dispositif peut comprendre deux ballonnets, chacun connecté à des moyens de gonflage qui lui sont propres.

La forme du ballonnet et sa taille ne sont pas limitatives de l'invention.

La présente invention, ses caractéristiques et les différents avantages qu'elle procure apparaîtront plus clairement à la lecture de la description détaillée qui suit d'un mode de réalisation, présenté à titre d'exemple explicatif et non limitatif, en référence aux figures annexées sur lesquelles :
- la Fig. 1 représente une vue en coupe transversale d'un mode de réalisation particulier des premiers et deuxièmes moyens de connexion étanche, ceux-ci étant montés sur l'abdomen d'un patient qui comporte deux stomies ;
- la Fig. 2 représente de manière schématique une vue de face d'un mode de réalisation particulier des moyens anti-reflux de l'invention ;
- la Fig. 3 représente de manière schématique une vue de dessus des moyens anti-reflux représentés sur la Fig. 2, le ballonnet étant en position d'ouverture ; et
- la Fig. 4 représente une vue de dessus des moyens anti-reflux représentés sur la Fig. 2, le ballonnet étant en position de fermeture.

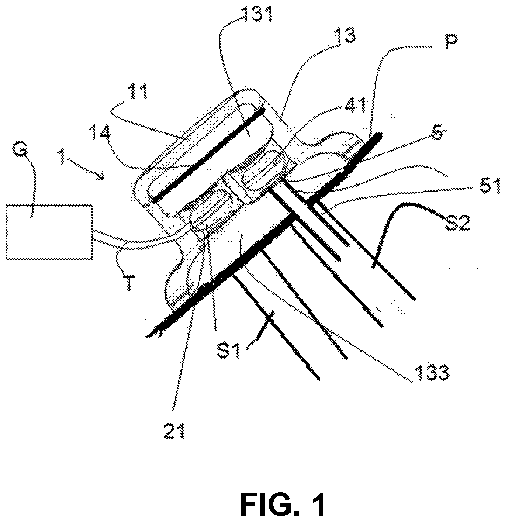

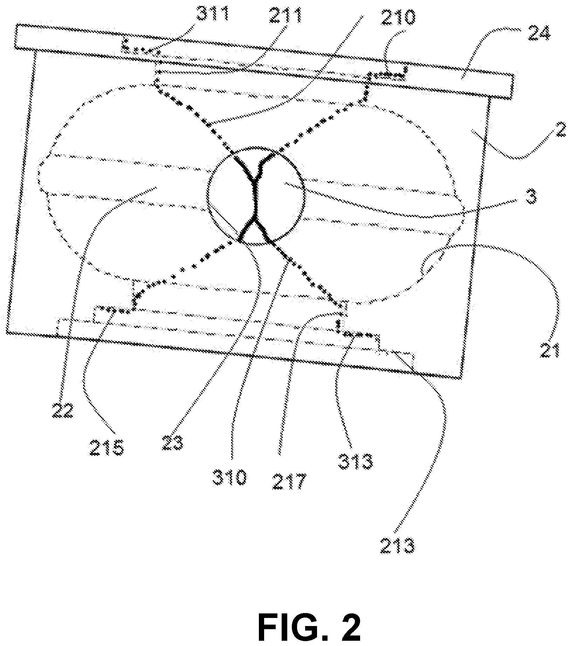

En référence à la Fig. 1, le dispositif comporte un collecteur unique 1 qui constitue les premiers et deuxièmes moyens de connexion étanche aux stomies amont et aval S1 et S2 qui sont pratiquées sur l'abdomen du patient. Ce dispositif est adapté pour des stomies rapprochées pouvant être recouvertes par un seul collecteur. Le collecteur 1 comporte un couvercle 11 fixé sur un corps de collecteur 13. Le corps de collecteur 13 présente un logement de membrane 131 qui contient une membrane 14. Un conduit d'aspiration 3 et un conduit de refoulement 5 sont ménagés dans le corps de collecteur 13 et débouchent dans le logement de membrane 131. Le corps de collecteur 13 comprend également un logement de collecte 133 dans lequel débouchent également les conduits d'aspiration 3 et de refoulement 5. Le logement de membrane 131 et le logement de collecte 133 sont reliés l'un à l'autre par les conduits d'aspiration 3 et de refoulement 5. Le logement de collecte 133 est fixé sur la peau P de l'abdomen du patient, au-dessus des deux stomies S1 et S2. Le conduit de refoulement 5 comprend une buse qui traverse le logement de collecte 133 et sur laquelle est connecté un tube 51. Ce tube 51 est inséré dans une portion d'intestin ouverte au niveau de la stomie aval S2. Le conduit d'aspiration 3 débouche directement dans le logement de collecte 133. Le conduit d'aspiration 3 comprend des premiers moyens anti-reflux 33 tandis que le conduit de refoulement comprend des deuxièmes moyens anti-reflux 53 qui seront tous deux décrits plus en détail en référence aux Fig. 2 à 4. Les deux conduits 3 et 5 ne communiquent pas directement entre eux et sont ménagés dans le corps de collecteur 13, entre le logement de membrane 131 et le logement de collecte 133. La cavité 21 est ménagée dans le conduit d'aspiration, tandis que la cavité 41 est ménagée dans le conduit de refoulement. Ces deux cavités 21 et 41 sont sensiblement identiques et présentent chacune une section ellipsoïdale dans un plan perpendiculaire aux conduits 3 et 5 ; la dimension la plus longue est perpendiculaire aux conduits 3 et 5, respectivement. Les deux cavités 21 et 41 sont formées dans des bagues cylindrique 2 montées de manière étanche dans les conduits 3 et 5, comme plus amplement expliqué en référence à la Fig. 2. Des moyens de gonflage G des ballonnets des premiers et deuxièmes moyens anti-reflux comportent des tubes de gonflage T qui sont connectés à ces ballonnets. Sur la Fig. 1, les ballonnets ne sont pas représentés par souci de clarté et un seul tube T a été représenté.

La Fig. 2 représente plus en détails un mode de réalisation des premiers moyens anti-reflux. Dans le mode de réalisation particulier ici représenté, les premiers et deuxièmes moyens anti-reflux sont identiques, mais l'invention n'est pas limitée à ce mode de réalisation.

En référence à la Fig. 2, les moyens anti-reflux comportent une bague 2 de surface externe sensiblement tubulaire et apte à être montée de manière étanche dans le conduit d'aspiration 3 ou dans le conduit de refoulement 5 du collecteur 1. Cette bague 2 comporte une cavité interne 21 de section maximale sensiblement circulaire. La cavité 21 présente une forme ellipsoïdale sensiblement semblable à celle d'un ballon de rugby. Une gorge 22 est ménagée sur toute la section de la cavité 21. La cavité 21 présente une perforation 23 sur laquelle est fixée l'extrémité du tube T (voir Fig. 1) qui est connecté aux moyens de gonflage et permet ainsi de gonfler plus ou moins le ballonnet. La gorge 22 débouche au niveau de la perforation 23.

La bague 2 présente un épaulement 24 qui vient en appui sur la surface du corps de collecte 13 au niveau de l'embouchure du conduit d'aspiration 3 ou de refoulement 5, dans la chambre de membrane 131. La cavité 21 est ouverte vers le haut et vers le bas de la bague 2. Un premier lamage 210 est pratiqué au niveau de l'extrémité supérieure de la bague 2, laquelle comprend l'épaulement 24. Ce lamage 210 se prolonge par un premier passage cylindrique 211 qui débouche dans la cavité 21. De même, à l'autre extrémité de la bague 2, se trouvent un deuxième lamage 213 qui se prolonge par un troisième lamage 215 de plus petite section. Ce lamage 215 se prolonge par un deuxième passage sensiblement cylindrique 217 qui débouche dans la cavité 21. La cavité 21, les lamages et les passages précités forment un passage pour le transit du bol alimentaire.

En référence à la Fig. 2, le ballonnet 3 est formé par une bande 310, cylindrique en matériau élastiquement déformable et utilisable dans le domaine médical pour un contact prolongé avec le corps humain. La bande 310 présente un bord haut 311 qui est fixé par collage sur la portion verticale du passage 211 et sur la portion horizontale du premier lamage 210, sur tout le périmètre de la section de ces derniers. Le bord inférieur 313 de la bande 310 est fixé par collage sur la portion horizontale du troisième lamage 215 et sur la paroi verticale du deuxième passage 217.

Sur la Fig. 2, le ballonnet 3 formé par la bande 310 est en position de fermeture. Il est gonflé par les moyens de gonflable G et les portions de bande 310 opposées selon un diamètre de la section de la cavité 21 sont en contact sur une portion de la hauteur de la bande 310. Le col 32 est écrasé, resserré sur lui-même selon le diamètre de la cavité 21 (c'est-à-dire la dimension sensiblement perpendiculaire à la hauteur de la bague 2) du fait du gonflage du ballonnet 3. Le ballonnet 3 occupe toute la section de la cavité 21, empêchant le passage du bol alimentaire à travers le col 32 qui est alors inexistant.

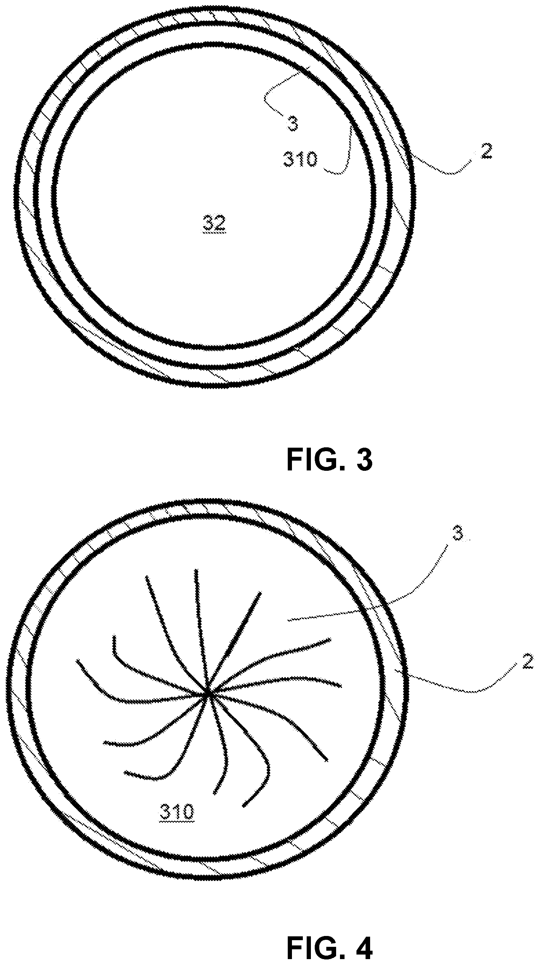

En référence à la Fig. 3, lorsque le ballonnet 3 est dégonflé et donc en position d'ouverture, la bande 310 se trouve à proximité de la paroi de la cavité 21 et forme un tore dont le col 32 (ou ouverture) permet le passage du bol alimentaire. Il est même possible en modulant le gonflage du ballonnet de coller la membrane 310 contre la paroi de la cavité 21, sous l'effet de l'aspiration générée par les moyens de gonflage G. La cavité est donc libre et permet le passage du bol alimentaire.

En référence à la Fig.4, on remarque que lorsque le ballonnet est gonflé, il n'y a plus de passage pour le bol alimentaire, le col 32 n'existe plus et s'est écrasé sur lui-même. La bande 310 vient en contact avec elle-même et du fait de la pression de fluide existant dans le ballonnet 3 assure une fermeture étanche du col 32 ; le bol alimentaire ne peut plus passer à travers la cavité 21.

Le fonctionnement du dispositif de l'invention va être expliqué plus en détails en référence aux Fig. 1 à 4.

Lorsque du bol alimentaire arrive dans l'intestin, en amont de la stomie amont S1, il est éjecté du fait des contractions intestinales dans le conduit d'aspiration 3. Il traverse la cavité 21 à travers le col 32 du ballonnet 3 et parvient dans la chambre de membrane 131. Les moyens de gonflage G gonflent le ballonnet 3 situé dans le conduit d'aspiration de manière à obturer le col 32. Les moyens formant pompe expulsent le bol alimentaire du fait de la déformation de la membrane 14 vers le conduit de refoulement 5, le ballonnet 3 équipant le conduit de refoulement étant dégonflé et donc en position d'ouverture. Le bol alimentaire traverse ainsi la cavité 41 et le tube de refoulement 5 ; il pénètre alors dans la portion d'intestin en aval de la stomie aval 2. Les moyens de gonflage G gonflent alors le ballonnet 3 situé dans la cavité 41 pour éviter le reflux du bol alimentaire vers le conduit de refoulement 5 et vers la chambre de membrane 131.

L'ouverture du ballonnet 3 situé dans la cavité 21 du conduit d'aspiration permet la montée du bol alimentaire collecté dans la chambre de collecte 133 vers la chambre de membrane 131 et le cycle recommence tel que précédemment exposé, jusqu'à ce que qu'il n'y ait plus de bol alimentaire dans la chambre de collecte 133.

Selon un autre mode de réalisation non représenté, le dispositif comporte deux collecteurs reliés par un tube. Le premier collecteur est disposé sur la stomie amont S1 tandis que le second est disposé sur la stomie aval S2.

Selon un autre mode de réalisation, non représenté, le dispositif comporte un capteur de pression monté de manière à mesurer la pression régnant dans les ballonnets 3. Ce capteur permet de détecter la présence de bol alimentaire en aval ou en amont du ballonnet 3 ; en effet, le bol alimentaire appuyant sur le ballonnet 3, il modifie la pression régnant dans ce dernier ce qui indique la présence du bol alimentaire dans le dispositif selon l'invention.

## Revendications

1. Dispositif permettant de créer un flux de bol alimentaire entre deux stomies (S1 ; S2), du type comportant :
- des moyens formant pompe (14, 131) qui présentent une ouverture d'aspiration et une ouverture de refoulement, qui sont adaptés pour aspirer un bol alimentaire à travers ladite ouverture d'aspiration et le refouler à travers ladite ouverture de refoulement, qui sont externes au corps du patient et aptes à être montés sur le corps d'un patient,
- des premiers moyens de connexion étanche (1) aptes à relier ladite ouverture d'aspiration à une stomie amont (S1) située sur la paroi abdominale d'un patient ;
- des deuxièmes moyens de connexion étanche (1) aptes à relier ladite ouverture de refoulement à une stomie aval (S2), disposée sur la paroi abdominale dudit patient ;
- des premiers et/ou deuxièmes moyens anti-reflux (21, 3 ; 41, 3) aptes à éviter le reflux du bol alimentaire aspiré ou refoulé par lesdits moyens formant pompe vers ladite ouverture d'aspiration et/ou de refoulement ;
**caractérisé en ce que** lesdits premiers et/ou deuxièmes moyens anti-reflux comportent :
- une cavité (21 ; 41) dans laquelle transit ledit bol alimentaire ;
- un ballonnet gonflable (3) monté dans ladite cavité ; et
- des moyens de gonflage (G) dudit ballonnet qui permettent de modifier le gonflement de ce dernier,
**en ce que** ledit ballonnet (3) est conformé pour passer d'une position d'ouverture dans laquelle ledit ballonnet (3) est partiellement gonflé et présente un col (32) qui permet le passage du bol alimentaire, à une position de fermeture dans laquelle ledit ballonnet (3) est davantage gonflé que dans ladite position d'ouverture et dans laquelle ledit ballonnet est au contact de la paroi de ladite cavité et ledit col (32) est obturé du fait de l'écrasement de la portion de paroi dudit ballonnet définissant ledit col (32) sur elle-même, sur au moins une portion de la hauteur dudit col (32).

2. Dispositif selon la revendication 1, **caractérisé en ce que** ledit ballonnet (3) est monté dans ladite cavité (21 ; 41) de manière à ce que dans ladite position d'ouverture, la paroi dudit ballonnet (3) reste en contact avec la paroi de ladite cavité (21 ; 41), moyennant quoi le bol alimentaire circule uniquement à travers ledit col.

3. Dispositif selon l'une des revendications 1 et 2, **caractérisé en ce que** ledit ballonnet (3) présente une forme sensiblement torique.

4. Dispositif selon l'une quelconque des revendications précédentes, **caractérisé en ce que** ledit ballonnet (3) est monté directement ou indirectement sur ladite paroi de ladite cavité.

5. Dispositif selon l'une quelconque des revendications précédentes, **caractérisé en ce que** lesdits premiers et/ou deuxièmes moyens anti-reflux comportent, en outre, une bague (2) qui comporte ladite cavité (21 ; 41), **en ce qu'**ils comportent également une bande (310) en matériau élastiquement déformable fixée de manière étanche sur la paroi de ladite cavité (21 ; 41) et formant ledit ballonnet gonflable (3).

6. Dispositif selon la revendication 5, **caractérisé en ce que** ladite bague (2) comporte une perforation (23) pour le passage d'un fluide provenant desdits moyens de gonflage (G) du ballonnet et une gorge (22) creusée dans l'épaisseur de la paroi de ladite bague (2) et qui débouche au niveau de ladite perforation (23).

7. Dispositif selon l'une quelconque des revendications précédentes, **caractérisé en ce que** lesdits premiers et/ou deuxièmes moyens anti-reflux font partie desdits premiers moyens de connexion étanche et/ou desdits deuxièmes moyens de connexion étanche.

8. Dispositif selon l'une quelconque des revendications précédentes, **caractérisé en ce que** ladite cavité (21 ; 41) et ledit ballonnet (3) sont conformés pour que dans ladite position d'ouverture, ledit ballonnet (3) est en contact avec la paroi de ladite cavité (21 ; 41).

9. Dispositif selon l'une quelconque des revendications précédentes, **caractérisé en ce que** lesdits moyens formant pompe comportent une membrane déformable (14) formant une coupelle **en ce que** ladite membrane (14) est apte à passer de manière réversible d'une première position dans laquelle la première face de ladite membrane forme la surface interne concave de ladite coupelle à une deuxième position dans laquelle ladite première face forme la surface externe convexe de ladite coupelle.

10. Dispositif selon l'une quelconque des revendications précédentes, **caractérisé en ce qu'**il comporte des moyens de détection de la présence de bol alimentaire dans lesdits premiers moyens de collecte (1) et notamment des moyens de détection de la présence de bol alimentaire qui comportent des moyens de détection d'une surpression dans le ballonnet équipant lesdits premiers moyens anti-reflux

## Patentansprüche

1. Vorrichtung zum Erzeugen eines Speisebreistroms zwischen zwei Stomata (S1; S2) von der Art, die Folgendes umfasst:
- Pumpenmittel (14, 131), die eine Saugöffnung und eine Auslassöffnung aufweisen, die so angepasst sind, dass sie einen Speisebrei durch die Saugöffnung ansaugen und ihn durch die Auslassöffnung ausleiten, die sich außerhalb des Körpers des Patienten befinden und zur Befestigung am Körper eines Patienten geeignet sind,
- erste dichte Verbindungsmittel (1), die geeignet sind, die Saugöffnung mit einem vorgelagerten Stoma (S1) zu verbinden, das sich an der Bauchwand eines Patienten befindet;
- zweite dichte Verbindungsmittel (1), die geeignet sind, die Auslassöffnung mit einem nachgelagerten Stoma (S2) zu verbinden, das an der Bauchwand des Patienten angeordnet ist;
- erste und/oder zweite Rückflussverhinderungsmittel (21, 3; 41, 3), die geeignet sind, den Rückfluss des von den Pumpenmitteln angesaugten oder ausgeleiteten Speisebreis zur Saug- und/oder Auslassöffnung zu verhindern;
**dadurch gekennzeichnet, dass** die ersten und/oder zweiten Rückflussverhinderungsmittel Folgendes umfassen:
- einen Hohlraum (21; 41), durch den der Speisebrei hindurchgeht;
- einen aufblasbaren Ballon (3), der im Hohlraum befestigt ist; und
- Mittel zum Aufblasen (G) des Ballons, die es ermöglichen, die Schwellung des Letzteren zu ändern,
dass der Ballon (3) so geformt ist, dass er aus einer geöffneten Stellung, in der der Ballon (3) teilweise aufgeblasen ist und einen Hals (32) aufweist, der den Durchgang des Speisebreis zulässt, in eine geschlossene Stellung übergeht, in der der Ballon (3) stärker aufgeblasen wird als in der geöffneten Stellung und in der der Ballon in Berührung mit der Wand des Hohlraums ist und der Hals (32) durch Zusammendrücken des Wandabschnitts des Ballons, der den Hals (32) definiert, auf sich selbst, über zumindest einen Abschnitt der Höhe des Halses (32) geschlossen wird.

2. Vorrichtung nach Anspruch 1, **dadurch gekennzeichnet, dass** der Ballon (3) so im Hohlraum (21; 41) befestigt ist, dass in der geöffneten Stellung die Wand des Ballons (3) in Kontakt mit der Wand des Hohlraums (21; 41) bleibt, wodurch der Speisebrei nur durch den Hals läuft.

3. Vorrichtung nach einem beliebigen der vorstehenden Ansprüche 1 und 2, **dadurch gekennzeichnet, dass** der Ballon (3) eine im Wesentlichen torische Form aufweist.

4. Vorrichtung nach einem beliebigen der vorstehenden Ansprüche, **dadurch gekennzeichnet, dass** der Ballon (3) direkt oder indirekt an der Wand des Hohlraums befestigt ist.

5. Vorrichtung nach einem beliebigen der vorstehenden Ansprüche, **dadurch gekennzeichnet, dass** das erste und/oder zweite Rückflussverhinderungsmittel ferner einen Ring (2) umfasst, der den Hohlraum (21; 41) umfasst, dadurch, dass sie auch ein Band (310) aus elastisch verformbarem Material umfassen, das an der Wand des Hohlraums (21; 41) dicht befestigt ist und den aufblasbaren Ballon (3) bildet.

6. Vorrichtung nach Anspruch 5, **dadurch gekennzeichnet, dass** der Ring (2) eine Lochung (23) für den Durchgang eines Fluids aus den Mitteln zum Aufblasen (G) des Ballons und eine in die Wanddicke des Rings (2) eingegrabene Kehle (22) aufweist, die zur Lochung (23) führt.

7. Vorrichtung nach einem beliebigen der vorstehenden Ansprüche, **dadurch gekennzeichnet, dass** das erste und/oder zweite Rückflussverhinderungsmittel Teil der ersten dichten Verbindungsmittel und/oder der zweiten dichten Verbindungsmittel ist.

8. Vorrichtung nach einem beliebigen der vorstehenden Ansprüche, **dadurch gekennzeichnet, dass** der Hohlraum (21; 41) und der Ballon (3) so geformt sind, dass der Ballon (3) in der geöffneten Stellung in Berührung mit der Wand des Hohlraums (21; 41) ist.

9. Vorrichtung nach einem beliebigen der vorstehenden Ansprüche, **dadurch gekennzeichnet, dass** die Pumpenmittel eine verformbare Membran (14) umfassen, die eine Schale bildet, dass die Membran (14) geeignet ist, umkehrbar von einer ersten Stellung, in der die erste Seite der Membran die konkave Innenfläche der Schale bildet, in eine zweite Stellung überzugehen, in der die erste Fläche die konvexe Außenfläche der Schale bildet.

10. Vorrichtung nach einem beliebigen der vorstehenden Ansprüche, **dadurch gekennzeichnet, dass** sie Mittel zum Erkennen des Vorhandenseins von Speisebrei in den ersten Sammelmitteln (1) und insbesondere Mittel zum Erkennen des Vorhandenseins von Speisebrei aufweist, die Mittel zum Erkennen eines Überdrucks im Ballon beinhalten, der die ersten Rückflussverhinderungsmittel ausrüstet.

## Claims

1. Device making it possible to create an alimentary bolus flow between two stomas (S1; S2), of the type comprising:
- pump-forming means (14, 131) which has a suction opening and a backflow opening, which are adapted to suction an alimentary bolus through said suction and opening, and flow it back through said backflow opening, which are external to the body of the patient and capable of being mounted on the body of a patient;
- first sealed connection means (1) capable of connecting said suction opening to an upstream stoma (S1) situated on the abdominal wall of a patient;
- second sealed connection means (1) capable of connecting said backflow opening to a downstream stoma (S2), arranged on the abdominal wall of said patient;
- first and/or second anti-reflux means (21, 3; 41, 3) capable of avoiding the reflux of the alimentary bolus suctioned or flowed back by said pump-forming means towards said suction and/or backflow opening;
**characterised in that** said first and/or second anti-reflux means comprising:
- a cavity (21; 41) wherein said alimentary bolus passes through;
- an inflatable balloon (3) mounted in said cavity; and
- means for inflating (G) said balloon which make it possible to modify the inflation of the latter,
**in that** said balloon (3) is shaped to pass from an opening position wherein said balloon (3) is partially inflated and has a collar (32) which makes it possible for the passage of the alimentary bolus, to a closing position wherein said balloon (3) is more inflated than in said opening position and wherein said balloon is in contact with the wall of said cavity and said collar (32) is blocked, due to the flattening of the wall portion of said balloon defining said collar (32) on itself, on at least one portion of the height of said collar (32).

2. Device according to claim 1, **characterised in that** said balloon (3) is mounted in said cavity (21; 41) such that in said opening position, the wall of said balloon (3) remains in contact with the wall of said cavity (21; 41), subject to which the alimentary bolus only circulates through said collar.

3. Device according to one of claims 1 and 2, **characterised in that** said balloon (3) has a substantially toroidal shape.

4. Device according to any one of the preceding claims, **characterised in that** said balloon (3) is mounted directly or indirectly on said wall of said cavity.

5. Device according to any one of the preceding claims, **characterised in that** said first and/or second anti-reflux means further comprise a ring (2) which comprises said cavity (21; 41), **in that** they also comprise a strip (310) made of elastically deformable material fixed sealed on the wall of said cavity (21; 41) and forming said inflatable balloon (3).

6. Device according to claim 5, **characterised in that** said ring (2) comprises a perforation (23) for the passage of a fluid coming from said inflation means (G) of the balloon and a groove (22) hollowed in the thickness of the wall of said ring (2) and which opens out at the level of said perforation (23).

7. Device according to any one of the preceding claims, **characterised in that** said first and/or second anti-reflux means form part of said first sealed connection means and/or said second sealed connection means.

8. Device according to any one of the preceding claims, **characterised in that** said cavity (21; 41) and said balloon (3) are shaped such that said opening position, said balloon (3) is in contact with the wall of said cavity (21; 41).

9. Device according to any one of the preceding claims, **characterised in that** said pump-forming means comprise a deformable membrane (14) forming a cup **in that** said membrane (14) is capable of passing reversibly from a first position wherein the first face of said membrane forms the inner concave surface of said cup to a second position, wherein said first face forms the convex outer surface of said cup.

10. Device according to any one of the preceding claims, **characterised in that** it comprises means for detecting the presence of alimentary bolus in said first collection means (1) and in particular means for detecting the presence of alimentary bolus which comprise means for detecting an overpressure in the balloon equipping said first anti-reflux means.
